# EUROPÄISCHE PATENTSCHRIFT

(11) **EP 2 712 634 B1**
(45) Veröffentlichungstag und Bekanntmachung des Hinweises auf die Patenterteilung: **08.06.2016**
(21) Anmeldenummer: 13005164.2
(22) Anmeldetag: 17.09.2007
(51) Int. Cl.: A61L 31/04, A61L 31/10, A61L 31/14

(54) **Medizinisches Implantat**
Medical Implant
Implant Médical

(43) Veröffentlichungstag der Anmeldung: 02.04.2014
(62) Teilanmeldung aus: 07800645.9
(73) Patentinhaber: Synergy Biosurgical AG, 6301 Zug (CH)
(72) Erfinder: Bähre, Wolf-Friedrich, 8700 Küsnacht (CH); Ruffieux, Kurt, 8800 Thalwil (CH)
(74) Vertreter: Lusuardi, Werther

(56) Entgegenhaltungen:
- EP-A1- 1 000 958
- WO-A2-2008/112912
- US-A- 5 290 281
- US-A1- 2006 095 138

## Beschreibung

Die Erfindung bezieht sich auf ein medizinisches Implantat gemäss dem Oberbegriff des Patentanspruchs 1.

Biokompatible, thermoplastische Materialien für osteosynthetische und ähnliche Verfahren für Befestigungszwecke an menschlichen oder tierischen Knochen zu verwenden ist eine bekannte Technik und wurde auf verschiedene Weisen versucht, z.B. mittels äusserer Wärmeapplikation analog zu einer Heissleimpistole (z.B. US Patent 5.290.281) oder durch Verflüssigen des Polymers mittels Ultraschallenergie gemäss der WO2006/002569. Diese Techniken sind allerdings mit Nachteilen behaftet: Die Erwärmung mittels äusserer Wärmequellen - analog zu einer Heissleimpistole - führt dazu, dass ein Implantat sehr schnell eingebracht werden muss um nicht wieder abzukühlen währenddem es die Verbindung mit dem Knochen eingeht, weil es typischerweise eine nur geringe Wärmekapazität aufweist, und nur im erweichten Zustand kann das thermoplastische Material z.B. in die Zwischenräume im Knochen eindringen. Sobald das Material wieder abgekühlt ist erfolgt keine weitere Verbindung mit dem Knochen. Auch das notwendige übermässige Erhitzen des thermoplastischen Materials - um eine vorzeitige Erstarrung zu verhindern - ist nachteilig, weil sowohl das Material als auch das (Knochen-) Gewebe dadurch geschädigt werden.

Aus der EP-B 0 696 185 ist ein polymeres, ein Chromophor enthaltendes medizinisches Implantat bekannt, welches mit Laserlicht beaufschlagt werden kann, so dass durch die absorbierte elektromagnetische Strahlung das gesamte Implantat einschliesslich der Oberfläche erweicht werden kann. Das dort erwähnte medizinische Implantat (als Gefässstent) wird mittels eines Katheters in den menschlichen Körper eingeführt. Nachteilig hier ist, dass das gesamte Implantat erweicht wird, so dass durch diese Strukturschwächung beispielsweise ein Einführen des Implantates unter Kraftaufwand verunmöglicht wird. Ferner ist das beschriebene Implantat aufgrund dieser oben erwähnten Form und dadurch, dass es im beschriebenen Design in seiner Ganzheit erweicht wird, ungeeignet um wie in der hier vorliegenden Erfindung eine Osteosynthese zu erreichen. Insbesondere benötigt das Implantat gemäss der EP-B 0 696 185 eine äussere Kraftanwendung (mittels Ballonkatheter) um das Implantat (ein Gefässstent) zu verformen.

Aus der US 5,163,960 BONUTTI ET AL. ist ein Verfahren zur Herstellung eines Implantats durch Erhitzten einer oder mehrerer Kunststoffkomponenten mit Laser (oder anderen Wärmequellen) zwecks gegenseitiger Verklebung oder Verbindung mit einem Metallimplantat bekannt. Dieses bekannte Verfahren ist nicht für die Chirurgie anwendbar, wo die Aufgabe besteht ein teilweise erweichtes Kunststoffimplantat in eine Knochenbohrung einzupressen, so dass der erweichte Kunststoff in die Unregelmässigkeiten der Knochenwand eindringen und einen Formschluss bewirken kann.

Aus der EP-A 1 000 958 ist ein biodegradierbares, absorbierbares, milchsäurebasiertes Material mit Formgedächtnis bekannt, welches durch Temperatureinwirkung in eine ursprüngliche Originalform zurückverwandelbar ist. Die Temperaturerhöhung dient nur dazu dern Material de Rückgewinnung der Originalform zu erlauben, d.h. eine Gestaltsänderung zu erzielen. Eine Erweichung des Materials wird weder offenbart noch wäre ein solche im Zusammenhang mit einem Gedächtnisform-Polymeren nützlich oder erstrebenswert. Das bekannte Material weist denn auch keinen Farbstoff auf, der eine effiziente Temperaturerhöhung des Materials erlauben würde.

Hier will die Erfindung Abhilfe schaffen. Der Erfindung liegt die Aufgabe zugrunde, ein medizinisches Implantat zu schaffen, welches bei der Bestrahlung mit elektromagnetischer Strahlungs-Energie nur teilweise an definierten Stellen erwärmt und erweicht werden kann, so dass das Implantat in eine Kavität, beispielsweise eine Knochenkavität einpressbar ist, sich der Geometrie der Kavität anpassen kann und in die das Implantat umgebenden Zwischenräume im Knochen eingepresst werden kann, so dass das Implantat nach der Erkaltung und Verfestigung des Polymers durch Formschluss verankert bleibt.

Das erfindungsgemässe medizinische Implantat kann in verschiedenartigen Implantatformen realisiert werden, insbesondere als Schraube, Stift, Clip, Stecker, Platte, Nagel, Spickdraht, Cage, Pedikelschraube (-nagel), Piercing, Hautverbindung, Medikamententräger, Genmaterialträger, Träger von bioaktiven Faktoren, (z.B. Wachstumsfaktoren, Knochenbildungsfördernde Substanzen Schmerzmitteln, usw.) Träger von anderen Implantaten, Dübel, Klammer, Perle, Zahnimplantat, Zahnwurzelimplantat, Schlauch, Rohr, Faden, Faden in Schlauch oder Rohr, Gewebe, Geflecht, Gestrick, Strumpf, Band, lose Fasern, Faserknäuel, Faserflocken, Granulat, Kette, Anker mit oder ohne Fadengleitöse.

Die Erfindung löst die gestellte Aufgabe mit einem medizinischen Implantat, welches die Merkmale gemäss dem Anspruch 1 aufweist.

Ein wesentlicher Vorteil der Erfindung besteht darin, dass auch während der Einbringung in den Knochen das Implantat weiter erwärmt werden kann und dennoch seine innere mechanische Stabilität behält.

Bei dem erfindungsgemässen medizinischen Implantat wird der Effekt benutzt, dass durch die elektromagnetische Strahlungsenergie Energie an die anregbaren Elektronen im Chromophor übertragen wird, welche in der Folge eine Erwärmung und eventuell ein Erweichen des Polymers in dieser Region verursacht, während nicht anregbare Regionen im Implantat nicht erwärmt oder erweicht werden.

Für die nachfolgenden, in der gesamten Beschreibung häufig verwendeten Begriffe gelten die folgenden Definitionen:
**Schmelzen** / **Aufweichen** / **Erweichen:** Unter Schmelzen, Erwelchen oder Aufweichen des Implantatmaterials im erfindungsgemässen Sinne wird die Erweichung des Implantats durch die durch die Strahlungsabsorption erzeugte Wärme verstanden, bis sich das vorher nicht in nützlicher Weise (typischerweise von Hand) plastisch verformbare Implantat im Körper mit moderater Kraftanwendung (typischerweise von Hand) verformen lässt und in erfindungsgemässer Weise verwenden lässt.

**Lichtleiter:** Unter einem Lichtleiter werden einerseits flexible oder starre optisch lichtleitende Strukturen verstanden, wie z.B. Glasfaserkabel, verspiegelte Hohlschläuche (z.B. auch Nanotubes) in welchen Licht geleitet wird und welche verwendet werden um die elektromagnetische Strahlung von der Quelle bis zum Implantat zu leiten. Andererseits kann auch das Implantat selbst als Lichtleiter und Licht-Diffusor dienen. Nach Eintritt in das Implantat wird das Licht durch das Implantat geleitet bis es zu der Stelle gelangt, wo die Erweichung des Polymers, zumeist an der Oberfläche, erreicht werden soll. Um das Licht durch den Lichtleiter im Implantat an die gewünschte Stelle zu leiten, kann der Lichtleiter im Implantat das Licht einerseits leiten, d.h. z.B. zur Spitze eines Pins, und dort verteilen, damit es nun an die Oberfläche des Pins gelangt, z.B. mittels Diffusion.

**Lichtleistung** / **Lichtdurchlässigkeit:** Generell wird hier von optisch durchsichtigen Implantaten ausgegangen, welche die elektromagnetische Strahlung analog z.B. zu Glas durchtreten lassen. Diese Lichtdurchlässigkeit kann auch spezifisch für die eingeleitete Strahlung sein und andere Wellenlängen können reflektiert oder absorbiert werden. In einigen Ausführungen der Erfindung kann es aber wünschenswert sein, dass Zonen im oder am Implantat das Licht streuen, um eine gleichmässige Verteilung des Lichts zu erzielen. Diese Streuwirkung kann durch Kristalle, Blasen, Risse, Phasengrenzen aller Art, Fremdkörper oder Pigmente aller Art, oder Beimischungen von Polymeren aller Art erzielt werden. Insbesondere seien auch keramische Substanzen, wie Partikeln aus Calciumphosphat erwähnt.

**Lichtquelle:** Als Lichtquelle kommen alle Quellen elektromagnetischer Strahlung in Frage, namentlich Glühbirnen, Dampfentladungslampen, Dioden, Halbleiter, Funken, Flammen, Sonnenlicht usw. Besonders bevorzugt werden Dioden und LASER-Lichtquellen, z.B. folgende Typen von Lasern:

### Lasertypen:

Laser werden als Energiequelle bevorzugt, da diese typischerweise nur wenige eng begrenzte Frequenzen von elektromagnetischer Strahlung emittieren. So können die Absorptionsspektren des (oder mehrer bis vieler) Chromophors (Chromophore), des nicht absorbierenden Anteils des Implantats sowie der Körperumgebung aufeinander abgestimmt werden. In einer bevorzugten Anwendung strahlt der Laser in einer vorzugsweise monochromatischen Frequenz weiche vom Implantat kaum, vom Chromophor stark und von der Umgebung wiederum kaum absorbiert wird. So können auch unterschiedliche Zonen mit unterschiedlichen Chromophoren im Implantat versehen werden und so spezifisch mit der jeweils bevorzugten Frequenz der elektromagnetischen Strahlung erwärmt werden.

Besonders bevorzugt wird eine oder mehrere Frequenzen der Strahlung, welche von dem Chromophor/Pigment im Polymer oder vom Licht-absorblerenden Polymer besonders gut absorbiert wird.

Es seien alle gängigen Lasertypen, Schwingungsmodi, gepulster oder continuous-wave Betrieb mit eingeschlossen und an sich möglich. Bevorzugt werden Diodenlaser des Infrarotbereiches oder des sichtbaren Bereiches. Es ist unter Umständen auch wünschenswert, polarisierte Strahlung zu verwenden, z.B. durch Verwendung von Polarisationsfiltem im Implantat oder an der Strahlungsquelle, oder z.B. durch bereits polarisiert erzeugte elektromagnetische Strahlung. Polarisation kann so als Selektionsmittel für die gezielte Erwärmung des Implantats verwendet werden, insbesondere bei Verwendung von bevorzugt mit polarisiertem Licht anregbaren Chromophoren.

Die bevorzugte Wellenlänge der elektromagnetischen Strahlung liegt im Bereich zwischen 260 und 3'000nm vorzugsweise im sichtbaren Bereich und nahen infraroten Bereich bis 1200nm. Andere Wellenlängen sind aber denkbar. Die Form des Lichtstrahls kann beliebig sein, im Querschnitt rund, oval, rechteckig, sternförmig, dreieckig, als Strahlenbündel usw.

Hier eine nicht abschliessende Liste mit verwendbaren Lasern:

| **Gas Laser** | **Wellenlänge(n)** |
|---|---|
| Helium-neon Laser | 632.8 nm (543.5 nm, 593.9 nm, 611.8 nm, 1.1523 µm, 1.52 µm, 3.3913 µm) |
| Argon Laser | 454.6 nm, 488.0 nm, 514.5 nm (351 nm, 457.9 nm, 465.8 nm, 476.5 nm, 472.7 nm, 528.7 nm) |
| Krypton Laser | 416 nm, 530.9 nm, 568.2 nm, 647.1 nm, 676.4 nm, 752.5 nm, 799.3 nm |
| Xenon Ion Laser | Verschiedene Wellenlängen von UV bis Infrarot |
| Nitrogen Laser | 1337.1 nm |
| Kohlendioxid-Laser | 10.6 µm, (9.4 µm) |
| Kohlenmonoxid-Laser | 2.6 - 4 µm, 4.8 - 8.3 µm |
| Excimerlaser | 193 nm (ArF), 248 nm (KrF), 308 nm (XeCl), 353 nm (XeF) |

| **Chemische Laser** | **Wellenlänge (n)** |
|---|---|
| Hydrogen Fluoride Laser | 2.7 to 2.9 µm |
| Deuterium Fluoride Laser | ∼3800 nm (3.6 to 4.2 µm) |
| COIL (Chemical oxygen-iodine Laser) | 1.315 µm |

| **Farblaser** | **Wellenlänge(n)** |
|---|---|
| Farblaser | 390-435 nm (stilbens), 460-515 nm (coumarin 102), 570-640 nm (Rhodamine 6G), und weitere |

| **Metall-Dampf Laser** | **Wellenlänge(n)** |
|---|---|
| Helium-cadmium (HeCd) Metalldampf-Laser | 441.563 nm, 325 nm |
| Hellum-Quecksilber (HeHg) Metalldampf-Laser | 567 nm, 615 nm |
| Hellum-selenium (HeSe) Metalldampf-Laser | bis zu 24 Wellenlängen zwischen Rot und UV |
| Copper vapor Laser | 510.6 nm, 578.2 nm |
| Gold vapor Laser 627 nm | 627 nm |

| **Feststoff Laser** | **Wellenlänge(n)** |
|---|---|
| Rubin Laser | 694.3 nm |
| Nd:YAG Laser | 1.064 µm, (1.32 µm) |
| Er:YAG Laser | 2.94 µm |
| YLF (Nd:YLF) Feststoff-Laser | 1.047 and 1.053 µm |
| Neodymium doped Yttrium orthovanadate (Nd:YVO₄) Laser | 1.064 µm |
| Neodymium doped yttrium calcium oxoborate Laser, Nd:YCa₄O(BO₃)₃ oder einfach Nd:YCOB | ∼1.060 µm (~530 nm bei der zweiten harmonischen) |
| Neodymium glass (Nd:Glass) Laser | ∼1.082 µm (Silikat-Gläser), ∼1.054 µm (Phosphat-Gläser) |
| Titanium sapphire (Ti:sapphire) laser | 650-1100 nm |
| Thulium YAG (Tm:YAG) Laser | 2.0 µm |
| Ytterbium YAG (Yb:YAG) Laser | 1,03 µm |
| Ytterbium doped glass laser (rod, plate/chip, and fiber) | 1 µm |
| Holmium YAG (Ho:YAG) Laser | 2.1 µm |
| Cerium doped lithium strontlum(or calcium) aluminium fluoride (Ce:LISAF, Ce:LICAF) | ∼280 - 316 nm |
| Promethium 147 doped phosphate glass (¹⁴⁷Pm⁺³:Glass) solid-state laser | 933 nm, 1098 nm |
| Chromium doped chrysoberyl (alexandrite) laser | Typisch 700 bis 820 nm |
| Erbium doped and erbium-ytterbium codoped glass lasers | 1.53-1.56 µm |
| Trivalent uranium doped calcium fluoride (U:CaF₂) solid-state laser | 2.5 µm |
| Divalent samarium doped calcium fluoride (Sm:CaF₂) laser | 708.5 nm |
| F-center laser. | 2.3-3.3 µm |

| **Halbleiter Laser** | **Wellenlänge(n)** |
|---|---|
| Semiconductor laser diode | 0.4-20 µm, je nach Material |
| GaN | 0.4 µm |
| AlGaAs | 0.63-0.9 µm |
| lnGaAsP | 1.0-2.1 µm |
| lead salt | 3-20 µm |
| (Vertical cavity surface emitting laser (VCSEL) | 850 - 1500 nm, je nach Material |
| Quantum cascade Laser | Infrarot |
| DPSS-Laser | UV-Infrarot |
| Hybrid Silicon Laser | Infrarot |

**Absorption von elektromagnetischer Strahlung:** Unter Absorption der elektromagnetischen Strahlung wird verstanden, dass das Implantat dort wo Absorption stattfindet, typischerweise (aber nicht zwingend) mindestens doppelt so viel der eingestrahlten Energie absorbiert als in den Zonen ohne Absorption. Typischerweise wird aber Faktor 5-1000 mal mehr Energie absorbiert wo der energie-absorbierende Bereich liegt, als dort wo das Implantat nicht absorbierend ist.

Als Absolutwert absorbiert das Implantat in der nicht absorbierenden Zone 0-10% der einstrahlenden Energie, die Zone mit Chromophor 50-100% der Energie, die restliche Energie verlässt das Implantat in die Umgebung.

**Chromophor:** Unter Chromophor werden Farbstoffe oder Pigmente verstanden, welche dem Polymer zugegeben werden, um die elektromagnetische Strahlung Im Polymer zu absorbieren und in Wärme umzuwandeln.

In einer speziellen Anwendung können auch Substanzen verwendet werden, welche dem Implantat beigefügt werden oder dieses beschichten und keine chromophoren Eigenschaften haben. Bei Einbringung in den Körper verändern sich die Substanzen aber durch Kontakt mit dem Körper, vorzugsweise als Reaktion auf den Gewebe-pH, als Reaktion mit Körpersalzen, der Körperfeuchtigkeit oder der Körpertemperatur und durch diese Reaktion verfärbt sich die Substanz und wird für die elektromagnetische Strahlung absorbierend. So erwärmt sich nur diejenige Zone am Implantat, welche mit dem Körper in Berührung kommt, da sich nur dort das Implantat verfärbt.

Allgemein seien folgende Chromophore und Pigmente explizit eingeschlossen:
Chlorophyll, Carbon Black, Graphit, Fluoreszein, Methylenblau, Indocyanine Grün, Eosin; Eosin Y (514 nm), Ethyleosin (532nm), Acridin, Acridin Orange, Kupfer Phthalocyanin, Chrom-Kobalt-Aluminium Oxid, Eisen Ammonium Citrat, Pyrogallol, Logwood Extrakt, Chlorphyllin-Kupfer Komplex, D&C Blue No.9, D&C Green No. 5, [Phthalocyaninato(2-)] Kupfer, D&C Blue No. 2, D&C Blue No.6, D&C Green No. 6, D&C Violet No. 2, D&C Yellow No. 10. Ein Spezialfall sind fluoreszierende Chromophore, welche unter Umständen das Licht nicht absorbieren, aber Licht abstrahlen, welches von der Umgebung, dem Polymer oder zusätzlich eingebrachtem Chromophor absorbiert wird. -

**Der Absorptionskoeffizient** des Polymers ist ebenso wie derjenige des Chromophors variabel und je nach Indikation einzustellen. Indocyanin beispielsweise hat einen Absorptionskoeffizienten von 20'000 mg⁻¹cm⁻¹. Der resultierende Absorptionskoeffizient des Chromophor hängt natürlich auch von der Konzentration im Implantat ab, ein typischen Bereich liegt zwischen 1000 und 1'000'000 mol⁻¹ cm⁻¹.

**Poröse Oberfläche:** Unter einer porösen Oberfläche wird eine Oberfläche verstanden welche geeignet ist, nach Kontakt mit der Körperoberfläche oder mit Körpersäften wie z.B. Blut, durch Bestrahlung mit elektromagnetischer Strahlung erwärmt zu werden. Dies geschieht dadurch, dass der Kontakt mit dem Körper das Implantat verunreinigt und es an der Stelle der Verunreinigung lichtabsorbierend wird. Vor Kontakt mit dem Körper hat diese spezielle Ausführung der Implantate keine oder nur geringe Tendenz durch elektromagnetische Strahlung erwärmt zu werden. Besonders geeignet für eine entsprechende Oberfläche sind rauhe, poröse, unebene, schwammartige Oberflächen, evtl. beschichtet mit hydrophilen, gut saugenden Materialien wie z.B. Calciumphosphaten, andere Keramiken, Gips, etc.. Alternativ können auch Strukturelemente angebracht werden, durch welche Körperflüssigkelten in das Innere des Implantats gesogen werden oder fliessen (z.B. Blut durch Kapillarkräfte) und in diesem dort Licht absorbieren. Durch die Verformung des Implantats beim Eindrücken in den Körper oder beim Schaffen der gewünschten Verbindung werden die von Körper stammenden chromophoren Strukturen mit der Implantatoberfläche vermischt, was den lokalen Erwärmungseffekt noch verstärkt. Als Besonderheit wurde hier ausserdem der überraschende Effekt erzielt, dass in geeigneter Wellenlänge auch die unmittelbare Umgebung des Implantats im Körper mit aufgewärmt wurde, da die Wellenlänge so gewählt wurde, dass die das Implantat berührenden Körpersäfte oder die berührende Körperoberfläche welche mit der porösen Oberfläche des Implantats interagiert die elektromagnetische Strahlung absorbiert. Durch geeignete Impulslänge und Wellenlänge (oder Kombination von Wellenlängen) lässt sich aber erreichen, dass nur die unmittelbare (<1mm) Umgebung erwärmt wird und somit kein relevanter Gewebeschaden auftritt. Diese Erwärmung, welche vorzugsweise 100°C, besser noch 56°C nicht Überschreitet erleichtert die erweichten Thermoplaste besser in die Zwischenräume der Körperoberfläche zu fliessen. Dieser Effekt kann auch in den oben und unten erwähnten anderen Ausführungen erzielt werden, wenn die verwendeten Frequenzen der elektromagnetischen Strahlung sowie die Pulsart, -frequenz und -dauer sowie die Energiemenge entsprechend gewählt werden. Die poröse Oberfläche, z.B. eine Calciumphosphatbeschichtung wird erfindungegemäss mit einem Chromophor kombiniert, als zusätzliche Schicht oder als Mischung.

**Reflektierend beschichtetes** Polymer: Unter einer reflektierenden Beschichtung wird eine Beschichtung des Polymers verstanden, welche ein Austreten der elektromagnetischen Strahlung verhindert, so dass das Licht im Polymer verbleibt und auch bei nur geringer Absorption (fakultativ auch ohne Chromophor) dieses zu erwärmen vermag. Die reflektierende Beschichtung kann aber auch in Kombination mit einem Chromophor verwendet werden und z.B. dessen Wirkung verstärken. In einer weiteren Ausführung kann das Implantat verspiegelt werden um einen vorzeitigen Austritt des Lichts aus dem Implantat zu verhindern, um das Licht beispielsweise zur Spitze des Implantats zu leiten. So wirkt die Verspiegelung zur Unterstützung der Lichtführung innerhalb des Implantats.

Als reflektierende Beschichtung (welche auch In das Polymer-Innere eingearbeitet werden kann) kommen alle Licht-reflektierenden Substanzen in Frage, insbesondere Metalle, hier wiederum insbesondere körperverträgliche Metalle wie Gold; Titan, Platin, Silber, Stahl und Verbindungen davon.

**Frequenzmodulation:** Um eine lokale Erwärmung des Implantats zu erreichen gibt es auch die Möglichkeit, im Implantat Substanzen oder optische Elemente einzubringen, welche zwar nicht wesentlich selbst elektromagnetische Strahlung absorbieren, aber die Eigenschaft haben, die Frequenz des Lichts zu verschieben, typischerweise Frequenzverdopplerkristalle oder -Vervielfacherkristalle. Das langwellige Licht gelangt dabei durch das Implantat ohne dieses wesentlich zu erwärmen bis zur Zone mit der frequenzverändernden (normalerweise verdoppelnden) Eigenschaft, erwärmt diese selbst und tritt zu einem gewissen Prozentsatz in einer kürzeren Frequenz aus dieser aus, vom Rest des Implantats wesentlich stärker absorbiert wird. Dieser Effekt kann sich auch mehrfach wiederholen. Typische Substanzen dafür sind nichtlineare optische Substanzen z.B. Lithiumniobat (LiNbO₃), Kaliumdihydrogenphosphat KDP, Beta-Bariumborat (ß-BaB₂O₄ oder BBO), Lithiumtriborat oder DAST (Diethylaminosulfur trifluoride). Analog können auch Phasenübergänge oder Grenzschichten im oder am Implantat integriert werden, welche denselben Effekt haben.

**Energie:** Die verwendete Energie um das Implantat genügend zu erwärmen hängt von seiner Grösse, Anwendung und den lokalen anatomischen Gegebenheiten ab. Typische durchschnittliche Leistung der Lichtquelle: Für kleine Pins oder Fixationselemente (Durchmesser 0,1-5mm): ca. 0,1-50 Watt, vorzugsweise 0,5-10 Watt. Für die Fixation von grossen Prothesen oder für das Füllen grosser Knochendefekte 1-2'000 Watt.

Die Spitzenleistung während einzeln applizierter Pulse kann 5 kW und mehr erreichen. Das Ziel besteht darin, ein Polymer mit einem zu erweichenden Volumen V mit einem Wechselstrom der Leistungsdichte P = 0,005 - 5 Watt/mm³ innert ca. 0,1 - 10 Sekunden zur Erweichung zu bringen.

Die so applizierte Energie E entspricht dabei ca. E = 0,0005 - 50 Watt*Sekunden/mm³.

Der Lichtleiter kann auch in eine Bohrung im Implantat eingeführt werden, welche das Implantat bis an die gegenüberliegende eingefärbte Schicht durchringt. Falls beispielsweise nur die eingefärbte Schicht aufschmelzen soll, kann die eingefärbte Schicht, an welcher der durch die Bohrung im Implantat geführte Lichtleiter mit seinem vorderen Ende anliegt, eine Schichtdicke vom 0.1 bis 0.5 mm aufweisen.

Das erfindungsgemässe medizinische Implantat gestattet die Lösung verschiedenartiger Aufgaben, wovon einige im Folgenden näher beschrieben werden.
**Aufgabe A: Selektives oder globales Erwärmen und Aufweichen oder Verflüssigen von medizinischen Implantaten mittels elektromagnetischer Strahlung, während der Implantation derselben.**
   Der Kern des Pins Ist so konzipiert, dass er sich nicht oder nur teilweise erwärmt und hart bleibt. Gleichzeitig kann dieser Kern als optisches Element dienen und das Licht innerhalb des Implantats weiterleiten. So kann der Pin nun in ein vorgebohrtes Loch gestossen werden, welches z.B. ein Untermass aufweisen kann und das erwärmte, welche Polymer wird in die Zwischenräume im Knochen gepresst. Nach Ausschalten der Lichtquelle kühlt das Polymer (der Thermoplast) aus und wird schnell (< 1-2 Minuten) hart, die mechanische Verbindung ist geschaffen.
**Aufgabe B: Selektive oder globale Erwärmung eines einen Thermoplasten umfassenden Implantates um eine Verformung während der Implantation denselben zu erreichen.**
   Dabei wird beispielsweise ein Pin in seinem Verlauf mit einer einen Farbstoff, eine Eigenfärbung oder eine Farbschicht enthaltenden Zone versehen und wiederum mit elektromagnetischer Strahlung beaufschlagt An der einen Farbstoff, eine Eigenfärbung oder eine Farbschicht aufweisenden Zone wird sich der Pin erwärmen. An dieser Stelle kann sich der Pin in einer gewünschten Weise verformen.
**Aufgabe C: Erreichung einer lokalen Fixation eines polymeren Implantates Im Körper.**
   Ein Pin wird durch ein geeignetes Herstellungsverfahren, z.B. mittels Spritzgiessen, mit einer Eigenspannung versehen. Durch das Erwärmen des gesamten Pins relaxiert der Thermoplast und der Pin wird kürzer und nimmt im Durchmesser zu, was zu einer Fixation im oder am umgebenden Gewebe führt.
**Aufgabe D: Erreichung einer lokalen Verbindung zwischen mehreren Implantaten durch Verschweissung derselben untereinander.**
   Diese besteht im Verbinden von zwei thermoplastischen Implantatteilen, welche separat in den Körper eingebracht werden können. Dabei muss gewährleistet sein, dass die benötigte elektromagnetische Strahlung durch beide (oder eine Velzahl) zu verbindende Implantatteile dringen kann, im typischen Falle ist hier ein Granulat gemeint. Nach dem Einbringen der beiden (oder der Vielzahl) Implantattelle wird die Lichtstrahlung eingeleitet, die Implantatteile erweichen an ihrer Kontaktstelle und können durch ein Aufbringen von Druck zusammengefügt werden. So kann aber z.B. auch ein Faden verklebt werden um auf einen Knoten zu verzichten.
**Aufgabe E: Klemmen oder Ummantelung von Weichteilen oder Knochen.**
   Es kann z.B. ein Magenband aus dem Implantatmaterial geformt werden (als offener Ring) und durch Lichtzufuhr verformbar gemacht und zu einem geschlossenen Ring verbunden werden. Analog lässt sich ein Polymerband auch als Cerclagematerial verwenden.
**Aufgabe F: Herstellung von Implantaten, welche nach ihrer Einführung in den Körper durch Durchschneiden des Implantatmaterials veränderbar sind.**
   Das hier verwendete und beschriebene Implantatmaterial kann auch dazu verwendet werden, Implantate herzustellen, welche selektiv mit Lichtstrahlung durchtrennt oder eröffnet werden können, so kann z.B. ein Faden auch mittels elektromagnetischer Strahlung Insbesondere mit grosser Intensität oder Licht und einer vorzugsweise kleinen "scharfen" Lichtquelle durchschnitten werden. Eingeschlagene oder eingeschmolzene Pins können so z.B. an der Knochenoberfläche abgeschnitten oder anmodelliert werden bis sie plan mit der Oberfläche der Knochens sind. Medikamententräger können so eröffnet und Wirkstoffe freigesetzt werden.
**Aufgabe G: Leitung des Lichts Im Implantat:** Das Licht soll Im Implantat zu der gewünschte Zone geleitet werden, wo die Erweichung erreicht werden soll, d.h. wo die lichtempfindliche Zone liegt. So kann auch in relativ homogen eingefärbten Implantaten eine selektive Erweichung erreicht werden, da sich das Implantat dort zuerst erwärmen wird, wo es am konzentriertesten ist.
**Aufgabe H: Thermoregulation:** Die elektromagnetische Strahlung soll das Implantat nicht übermässig erhitzen und in der gewünschten Zone gleichmässig in Wärme umsetzen. Die Temperatur sollte, nicht über 500°C, vorzugsweise nicht über 250°C, Idealerweise unter 100°C liegen. Die Steuerung der Temperatur kann Ober das Chromophor erzielt werden, welches sich über einer kritischen Temperatur verfärbt oder entfärbt und somit die eingestrahlte Energie nicht mehr oder gar nicht mehr absorbiert (sog. "Thermochrome Chromophore"). Andererseits kann eine homogenere Temperaturverteilung durch eine gepulste Energieeinbringung erzielt werden, In den Phasen ohne Puls hat die Energie Zeit sich durch Wärmeleitung im Implantat zu verteilen. Eine andere Möglichkeit besteht darin, die lokale Temperatur zu messen und die Leistung entsprechend anzupassen. Die Messung kann mittels Temperatursensor, Infrarotmessung mittels Kamera oder Lichtleiter, Rutherford Backscattering erfolgen. Im speziellen kann der Lichtleiter und das Implantat selbst verwendet werden um die Wärmestrahlung zu messen, das heisst in umgekehrter Richtung als wie die elektromagnetische Strahlung eingespelst wird, es sind dazu geeignete optische Elemente wie halbdurchlässige Spiegel im Stahl oder andere dem Spezialisten bekannte Verfahren möglich. Die lokale Überhitzung lässt sich auch mittels lokaler oder genereller Kühlung, durch Luft, Flüssigkeit oder Isolationszonen (z.B. Zonen mit luftgefüllten Blasen oder mit Keramikpartikeln als Isolator oder ähnliches) erreichen. Dazu müssen unter Umständen entsprechende Kühlkanäle im Implantat vorhanden sein.

In einer speziellen Ausführungsform besteht das Implantat mindestens zum Teil aus einem zu erwärmenden Polymer, welches eine minimale molare Wärmekapazität cₚ von 1 ,6 kJ/kmolK, vorzugsweise von 2,2 kJ/kmolK aufweist.

Bei ein anderen Ausführungsform besteht das Implantat mindestens zum Teil aus einem zu erwärmenden Polymer, welches eine maximale molare Wärmekapazität cₚ von 2,9 kJ/kmolK, vorzugsweise von 2,5 kJ/kmolK aufweist.

Das Polymer sollte zweckmässigerweise derart ausgewählt ist, dass die Erweichung unterhalb einer Erwärmungstemperatur von 250°C stattfindet. In einer weiteren Ausführungsform findet die Erweichung unterhalb einer Erwärmungstemperatur von 150°C, vorzugsweise unterhalb von 100°C statt.

Bei einer weiteren Ausführung sind ausser dem Polymer selbst keine anderen Bauteile des Implantats für die Erwärmung des Implantats vorgesehen.

Vorzugsweise umfasst das Implantat Mittel zur Befestigung eines Lichtleiters mit mindestens einer lichtdurchlässigen Faser. Die Mittel können aus einer Vertiefung in oder einer Erhebung an der Oberfläche des Polymers bestehen.

Bei einer speziellen Ausführungsform ist das zu erwärmende und zu erweichende Polymer optisch und/oder mechanisch isotrop. Bei einer anderen Ausführungsform ist das zu erwärmende und zu erweichende Polymer optisch und/oder mechanisch anisotrop.

Das zu erwärmende und zu erweichende Polymer ist vorzugsweise ein thermoplastisches Material, welches aus den folgenden Gruppen gewährt ist: Poly-alpha-hydroxyester, Polyorthoester, Polyanhydrid, Polyphosphazenes, Poly(propylenfumarat), Polyesteramid, Polyethylenfumarat, Polylaktid, Polyglykolid, Polycaprolacton, Trimethylencarbonat, Polydioxanon, Polyhydroxybutyrat, sowie deren Copolymere und Gemische.

Bei einer speziellen Ausführungsform weist das zu erwärmende und zu erweichende Polymer eine matte oder optisch streuende offenporige Struktur auf.

Vorzugsweise weist das zu erwärmende und zu erweichende Polymer kapillare Kanäle auf.

Bei einer anderen Ausführungsform weist das zu erwärmende und zu erweichende Polymer hydrophile Eigenschaften auf.

Bei einer speziellen Ausführungsform umfasst das zu erwärmende und zu erweichende Polymer eine Mischung aus mindestens zwei verschiedenen körperverträglichen, thermoplastischen Materialien.

Das erfindungsgemässe medizinische Implantat eignet sich insbesondere zur Fixierung von Knochen oder Knochenfragmenten.

Neben dem einen Farbstoff enthaltenden Polymer kann das medizinische Implantat noch Implantatteile aus weiteren Materialien umfassen, vorzugsweise aus folgenden Gruppen ausgewählt: Metalle, Carbon, Keramik, PEEK, nicht thermoplastische Polymere, welche vorzugsweise aus der Gruppe der Polymethylmethacrylate ausgewählt werden und/oder anorganische Materialien, so wie Kalziumphosphat, Kalziumsulfat oder Knochenzement.

In einer weiteren Ausführungsform liegt das zu erwärmende und zu erweichende Polymer in Form einer Implantatbeschichtung vor.

In wiederum einer anderen Ausführungsform ist nur ein Teil der Oberfläche des Implantates mit dem zu erwärmenden und zu erweichenden Polymer beschichtet.

In einer anderen Ausführungsform umfasst das das zu erwärmende und zu erweichende Polymer Zonen mit einem unterschiedlichen Absorptionskoeffizienten a, insbesondere in Form von Oberflächenbeschichtungen.

In einer weiteren Ausführungsform weist die Beschichtung eine variable Schichtdicke auf.

In einer anderen Ausführungsform ist der spektrale Absorptionskoeffizient a des Materials mit niedrigem Absorptionskoeffizienten "a" kleiner als 1'000 Mol⁻¹cm⁻¹ , vorzugsweise kleiner als 100 Mol⁻¹cm⁻¹.

In wiederum einer weiteren Ausführungsform weist das medizinische Implantat eine feste Form auf.

In einer anderen Ausführungsform liegt das zu erwärmende und zu erweichende Polymer in Granulatform vor.

In einer weiteren Ausführungsform ist das medizinische Implantat aus Fasern hergestellt, wobei das zu erwärmende und zu erweichende Polymer vorzugsweise als Beschichtung der Fasern dient.

In wiederum einer weiteren Ausführungsform liegt das medizinische Implantat in Form eines offenporigen Schaums oder Schwammes vor.

In einer anderen Ausführungsform ist das medizinische Implantat als Knochenfixationselement ausgebildet, vorzugsweise in Form einer Knochenschraube, Knochenstift, Knochendübel, Pin, Platte, Dübel, Schlauch (Rohr), Faden, Faden in Schlauch/Rohr oder Anker (mit Fadengleitöse).

In einer weiteren Ausführungsform ist das medizinische Implantat als Zahnimplantat oder Zahnwurzelimplantat ausgebildet.

In wiederum einer weiteren Ausführungsform befindet sich das zu erwärmende und zu erweichende Polymer mindestens zum Teil in einem erweichten Zustand.

In einer anderen Ausführungsform ist der erweichte Zustand durch eine das Polymer durchdringende elektromagnetische Strahlung, vorzugsweise Licht der Wellenlänge 400 - 1300 nm oder Laserlicht erzeugt.

In einer weiteren Ausführungsform weist das Polymer keine gleichmässige Lichtdurchlässigkeit auf, und letztere ist vorzugsweise an der Oberfläche des Implantats kleiner als im Inneren des Implantats. Dadurch ist der Vorteil erreichbar, dass durch die vorzugsweise innere Ausleuchtung des Implantates erreicht wird, dass das Implantat selbst gleichzeitig als mechanische Stütze und Stabilisator, und andererseits als Lichtleiter verwendet wird.

Eine mögliche Vorrichtung zur Fixierung von Knochen oder Knochenfragmenten umfasst eine Knochenplatte, mit einem oder mehreren das Implantat durchquerenden Löchern und mindestens einem zur Einführung in die Löcher geeigneten erfindungsgemässen medizinischen Implantat, wobei das erfindungsgemässe medizinische Implantat im nicht erweichten Zustand gegenüber den Löchern überdimensioniert ist.

Der Farbstoff für das erfindungsgemässe medizinischen Implantates kann mittels einer der folgenden verschiedenen Varianten in ein Polymer eingearbeitet werden:
1) Als vorteilhaft erweist sich ein Compoundieren, bei welchem der Farbstoff in die Schmelze eingearbeitet und durch Misch- und Scherprozesse im Polymer homogen verteilt wird. Mittels solchen Compounds können direkt erfindungsgemässe Implantate oder Implantatteile im Spritzgussprozess hergestellt werden.
   Verlangt die Anwendung das Vorhandensein von farbstoffhaltigen Polymerschichten oder Implantatteilen, so können diese im sogenannten Zweikomponenten-Spritzgussverfahren hergestellt werden. Dabei wird in einem ersten Schritt das ungefärbte Teil des Implantates gespritzt und nach einer Veränderung der Kavität in der Spritzgussform in einem zweiten Schritt der farbstoffhaltige Teil gespritzt.
2) Die Schichten von farbstoffhaltigem Polymer werden über ein Aufbringen und Trocknen von farbstoff- und polymerhaltigen Lösungen erreicht. Dabei können Schichten von farbstoffhaltigem Polymer über das Abscheiden und Trocknen von farbstoff- und polymerhaltigen Lösungen erreicht werden, analog dem Prinzip des Kerzenziehens (Dipcoating Prozess) oder durch ein Aufspritzen. Mittels dem vorgängig beschriebenen Abscheideverfahren können sehr dünne (Mikrometerbereich) bis dicke Schichten (sub- und Millimeterbereich) erreicht werden.
3) Die mindestens eine Farbschicht wird über ein Auftragen und Trocknen einer farbstoffpartikelhaltigen Suspension oder Lösung erreicht.
4) Die Beschichtung kann durch folgende Schritte erfolgen:
   a) Erhitzen von farbstoffhaltigen Partikeln;
   b) Aufschiessen der erhitzten Partikel auf die Oberfläche des ungefärbten Teils des medizinischen Implantates, so dass die Partikel das Polymer des ungefärbten Teils des medizinischen Implantates aufschmelzen und so in der Oberfläche fixiert werden.

Keramische oder andere nicht thermosensitive Partikel können auf die Oberfläche gebracht werden, in dem diese in einem erhitzten Zustand auf die Polymeroberfläche geschossen werden, dort lokal das Polymer aufschmelzen und somit in der Oberfläche fixiert werden. Als Beispiel hierfür dient der Plasmasprühprozess mit welchem z.B. Hüftgelenkprothesen mit Calciumphosphat-Partikeln beschichtet werden. Prozesse wie Chemical Vapor Deposition (CVD) oder Physical Vapor Deposition (PVD) sind bei geeigneten Substraten denkbar.

Die oben genannten Verfahren sind auch denkbar zum Aufbringen von reflektierenden Schichten. Hierbei werden die reflektierenden Stoffe in ein Polymer eingearbeitet, mittels Abscheiden einer Lösung oder Suspension oder mittels einem direkten Auftragen oder Einschmelzen auf die Oberfläche gebracht.

In einer anderen Ausführungsform eines möglichen Verfahrens zur Herstellung eines erfindungsgemässen medizinischen Implantates kann das Polymer derart ausgewählt, dass die Erweichung oberhalb einer Erwärmungstemperatur von 40°C stattfindet.

Bei einer Anwendung des erfindungsgemässen Implantats zur Osteosynthese kann das medizinische Implantat im nicht erweichten Zustand gegenüber der Knochenbohrung entweder überdimensioniert oder unterdimensioniert sein. Das zu erwärmende Polymer kann in Form einer Stange durch einen Hohlraum eines Implantates eingeführt werden oder durch ein hohles Instrument.

Das zu erwärmende und zu erweichende Polymer kann auch in ein einen Hohlraum mit radialen Austrittslöchern aufweisendes Implantat eingeführ werden.

Im Folgenden werden die Verfahrensschritte bei der Verwendung eines erfindungsgemässen medizinisches Implantates näher beschrieben:
a) Vorbereitung des Knochens, z B. durch Einbringen eines Bohrlochs;
b) Ansetzen des gegenüber dem Bohrloch überdimensionierten Fixationselementes in das Bohrloch;
c) Erwärmen des (thermoplastischen) Polymers des Implantats mittels Bestrahlung durch Licht;
d) Eindrücken/Einpressen des teilverflüssigten Implantats/Pins in die Kavität unter Ausfüllung der diversen Hohlräume und ihrer ev. Verästelungen; und
e) Abkühlen und verfestigen lassen des Implantats, was z.B. mittels aktiver Kühlung unterstützt werden kann.

Ein Verfahren zur Osteosynthese kann folgende Schritte umfassen:
Positionieren eines erfindungsgemässen medizinischen Implantates ausserhalb einer Implantationsstelle;
Erwärmen des Polymers des erfindungsgemässen medizinischen Implantates mittels elektromagnetischer Strahlung bis zur Aufweichung des Polymers; und
Einführen des mindestens teilweise aufgeweichten erfindungsgemässen medizinischen Implantates in die Implantationsstelle.

Das erfindungsgemässe medizinische Implantat eignet sich insbesondere zur Fixierung von Knochen oder Knochenfragmenten und speziell für die Vertebroplastik. Das erfindungsgemässe medizinische Implantat eignet sich im Weiteren für die Verriegelung und/oder Zentrierung von Implantaten, insbesondere Marknägeln nach deren Einbringung in den Knochen.

In einer anderen Ausführungsform des Verfahrens zur Osteosynthese ist das medizinische Implantat im nicht erweichten Zustand gegenüber der Knochenbohrung nicht überdimensioniert und weist eine innere Vorspannung auf.

In einer weiteren Ausführungsform des Verfahrens zur Osteosynthese wird das zu erwärmende Polymer in Form einer Stange durch einen Hohlraum eines Implantates eingeführt oder durch ein hohles Instrument.

In wiederum einer weiteren Ausführungsform des Verfahrens zur Osteosynthese wird das zu erwärmende und zu erweichende Polymer In ein einen Hohlraum mit radialen Austrittslöchem aufweisendes Implantat eingeführt.

Im Folgenden werden die Verfahrensschritte bei der Verwendung eines erfindungsgemässen medizinisches Implantates näher beschrieben:
a) Vorbereitung des Knochens. Z.B. Einbringen eines Bohrlochs;
b) Ansetzen des gegenüber dem Bohrloch überdimensionierten Fixationselementes in das Bohrloch;
c) Erwärmen des (thermoplastischen) Polymers des Implantats mittels Bestrahlung durch Licht;
d) Eindrücken/Einpressen des teilverflüssigten Implantats/Pins in die Kavität unter Ausfüllung der diversen Hohlräume und ihrer ev. Verästelungen; und
e) Abkühlen und verfestigen lassen des Implantats, was z.B. mittels aktiver Kühlung unterstützt werden kann.

Ein Verfahren zur Osteosynthese kann folgende Schritte umfassen:
Positionieren eines erfindungsgemässen medizinischen Implantates ausserhalb einer implantationsstelle;
Erwärmen des Polymers des erfindungsgemässen medizinischen Implantates mittels elektromagnetischer Strahlung bis zur Aufweichung des Polymers; und
Einführen des mindestens teilweise aufgeweichten erfindungsgemässen medizinischen Implantates in die Implantationsstelle.

Das erfindungsgemässe medizinische Implantat eignet sich insbesondere zur Fixierung von Knochen oder Knochenfragmenten und speziell für die Vertebroplastik. Das erfindungsgemässe medizinische Implantat eignet sich im Weiteren für die Verriegelung und/oder Zentrierung von Implantaten, insbesondere Marknägeln nach deren Einbringung in den Knochen.

### Beispiel 1 (Plattenosteosynthese)

Eine resorbierbare Osteosyntheseplatte mit einer Dicke von 1 mm aus einem Poly-D, L-Lactid wurde auf die zu fixierenden Knochenfragmente aufgebracht und die benötigten Löcher in den Knochen gebohrt. In diesem Beispiel handelte es sich um eine Platte mit Löchern für 2.0 mm Schrauben. Es wurden Löcher von 1.7 mm in den Knochen gebohrt. Anschliessend wurde ein teilweise lichtleitender Pin mit einem 2.0 mm Durchmesser durch das Schraubenloch in der Platte auf des vorgebohrte Loch aufgesetzt und mit Licht beaufschlagt (Leistung 3 Watt, Wellenlänge 808nm). Die Energie des Lichts floss durch den lichtleitenden Pin und erwärmte diesen in der Zone, welche mit Carbon Black eingefärbt wurde. Durch sanften Druck auf den Pin konnte nun der Pin in das in den Knochen vorgebohrte Loch geschoben werden und das thermoplastische Material floss In die zugänglichen intertrabekulären Zwischenräume im sponglösen Knochen. Nach Ausschalten der Lichtquelle kühlte das Polymer wieder ab und erstarrt in weniger als 1 Minute aus. Der mit einem etwas überdimensionierten (d.h. breiter als die Plattenbohrung) Kopf versehen Pin hielt nun die Platte an der gewünschten Stelle.

### Beispiel 2 (Plattenosteosynthese)

Bei einer Variante des Beispiels 1 wurde eine Knochenplatte verwendet, welche ebenfalls aus dem gleichen Polymer wie der oben beschriebene Pin gefertigt wurde. Der Pin wurde analog zum obigen Beispiel eingebracht. Sobald der Kopf des Pins in Kontakt mit der Platte geriet fand hier ebenfalls eine Verschmelzung zwischen Platte und Pin statt da die Platte im Bereiche des Loches ebenfalls lichtabsorbierend ist und dort ein Verschmelzen von Platte und Kopf erreicht wurde. Nach dem Abkühlen waren Pin und Platte fest miteinander verbunden, die Verbindung wurde dadurch winkelstabil.

### Beispiel 3 (Knochenanker)

Die zu lösende Aufgabe war hier die Fixation eines Fadens im Knochen, um mit dem Faden z.B. eine Sehne oder ein anderes Knochenstück zu halten. Dafür wurde ein Loch mit einem Durchmesser von 3 mm bis 15 mm tief in den Knochen gebohrt. In das Loch im Knochen wurde ein Faden eingeführt, welcher einen hohen Schmelzpunkt aufweist. Auf das Loch wurde danach ein Anker aufgesetzt welcher etwas dicker als das Loch war.

Analog zum Beispiel 1 wurde auch hier der Anker mittels Diodenlicht mit Energie beaufschlagt und nach Aufweichen durch die Strahlungsenergie in den Knochen gedrückt. Nach Ausschalten der Lichtquelle, erstarrte das Polymer und der Anker hielt im Knochen und mit Ihm der Faden.

### Beispiel 4 (Knochenanker)

Bei einer Abwandlung des Beispiels 3 wurde der Faden durch eine Querbohrung im Anker geführt, der Anker anschliessend in den Knochen geführt und dort mit einer Glasfaser-Lichtquelle gehalten (gespiesen von einer Lampe oder einem Laser). Anschliessend wurde die abgerissene Sehne mittels des Faden befestigt. Hierbei wurde der Faden unter einer Zugkraft gehalten. Durch das gleichzeitig erfolgte Einschalten des Lichts schmolz der Anker teilweise auf und verklebte unter etwas Druck mit dem Faden und gewann so Halt im Knochen. Nach dem Abkühlen innert ca. 30 Sekunden konnte dann die Zugkraft auf dem Faden gelöst werden. Das sonst notwendige Verknoten des Fadens erübrigte sich.

### Beispiel 6 (Implantation einer Prothese)

Bei einem Zahnimplantat aus Titan wurde das distale Drittel mit einem partiell lichtabsorbierenden Polymer umgeben. Das Implantat selbst wurde so gefertigt, dass es lichtleitend Ist (mit Kanälen versehen, von der von der Wurzelspitze weggewandten Seite zum Polymer hin). Dort wurde die Lichtquelle angeschlossen. Das Implantat wurde in das mit Untermass vorgebohrte Loch gesetzt und das Licht eingeschalten. Das Polymer wurde vom Licht erwärmt und das Implantat konnte In den Zahnwurzelkanal geschoben werden. Das Erstarren des Polymers nach Ausschalten des Lichts im Knochen führte zu einer primären, belastungsstabilen Verbindung zwischen Knochen und Implantat. Die Beschichtung aus Polylactid-co-glykolid degradiert innert wenigen Tagen und erlaubt danach ein Anwachsen von Knochen an das Titanimplantat.

### Beispiel 6 (Gefässclip)

Der Clip diente zum Abklemmen von Blutgefässen zur Blutstillung, Er bestand im Wesentlichen aus zwei Schenkeln und einem Scharnier. Die Schenkel wurde mit einer Klemme gegriffen und das Blutgefäss dazwischen gehalten. Die Schenkel wurden unter Lichtzufuhr zusammengedrückt. An der Berührungsstelle der Schenkel und am Scharnier ist das Implantat lichtabsorblerend, ansonsten lichtleitend. Durch die Klemme wurde das Licht an die Berührungsstelle und zum Scharnier geleitet. Das Licht erweichte dadurch das Scharnier und eriaube ein Verbiegen des Clips. Beim Aufeinandertreffen der dem Scharnier abgewandten Enden der Schenkel erfolgte hier eine Verklebung der beiden Schenkel.

### Beispiel 7 (Verspiegelung)

Ein Pin aus Poly-D, L-Lactid mit glatter Oberfläche von 7mm Länge und 2.5mm Durchmesser wurde am Kopf an eine Lichtquelle angeschlossen und in ein vorgebohrtes 1.5mm Loch geführt. Das Licht wurde am Kopf des Pins eingeleitet und In zu der Pin-Spitze gerichtet Im Bereiche der Pin-Spitze (weggewandt vom Kopf des Pins) ist das Implantat mit Gold bedampft und beschichtet worden (Schichtdicke < 0.1 mm In dieser Ausführung). Die elektromagnetische Strahlung wurde darauf an der verspiegelten Oberfläche des Pins nach innen reflektiert und zurückgeworfen. Obwohl das Pin-Material diese Strahlung zwar kaum absorbiert, reichte die geringe Absorption von <30% dazu aus, die mehrfach reflektierte Strahlung aufzunehmen und den Pin lokal zu erwärmen. Dieser schmolz dadurch auf und konnte in den Knochen (d.h. die Bohrung) gedrückt werden. Das geschmolzene Polymer drang in die intertrabekulären Räume ein und fand so nach Abschalten des Lichts und nach Auskühlung dort festen Halt.

Im Laboratorium konnte nachgewiesen werden, dass die Lichtenergie durch die verspiegelungs-bedingte verstärkte Absorption im Polymer und nicht durch die Spiegel-Schicht selbst erzeugt wurde, indem eine Beleuchtung des Implantats von aussen keine Erweichung des Polymers erzielen konnte, nur durch Bestrahlung in das Innere des Polymers durch eine nicht-verspiegelte Zone konnte oben beschriebener Effekt erzielt werden. Es sind aber auch Ausführungsformen denkbar, wo eine partielle Verspiegelung und partielle Strahlungsabsorption erfolgen.

Ein besonderer Vorteil der Verspiegelung ist ferner, dass sobald die Oberfläche des Polymers schmilzt und sich verformt, die Reflexion nachlässt und somit auch die weitere Erwärmung gebremst wird. So kann eine lokale Überhitzung vermieden werden.

### Beispiel 8 (Vertebroplastik)

In eine osteoporotische Kompressionsfraktur eines Lendenwirbels wurde durch die Pedikel (in Lokalanästhesie) von dorsal ein Loch von 4mm Durchmesser in den Wirbelkörper gebohrt (Länge ca. 4cm). Durch das Loch wurde von dorsal noch ohne Lichteinwirkung ein partiell eingefärbter Poly-D-L-lactid Pin eingeführt (Durchmesser 3.9mm), versetzt mit Carbon Black und einer dadurch resultierenden Lichtabsorption von 85% an der Oberfläche. Nun wurde die Lichtquelle eingeschaltet und der Pin in den Wirbelkörper geschoben. Unter Nachschieben des Pins konnte so eine Füllung des Wirbelkörpers mit dem Poly-D-L-lactid erreicht werden. Nach 2 Minuten auskühlen war der Wirbelkörper belastungsstabil.

### Beispiel 9 (Defektfüllung)

Derselbe Pin wie in Beispiel 9 beschrieben wurde auch zum Füllen des Defektes von Knochen verwendet, hier zum Füllen eines Tiblakopfdefekts. Dafür wurde bei einem Patienten mit Tibiakopffraktur ein 4mm Loch von ventral durch die Kortikalis zum Defekt hin gebohrt. (Länge 2cm). Durch dieses Loch wurde darauf der Pin in die Markhöhle und den sponglösen Raum des Knochens unter Lichtanwendung geschoben, und so wie bei einer Verbundosteosynthese ein stabiler Knochen geschaffen. Die danach in dieses Gebiet eingebrachten Schrauben hielten im geschmolzenen Polymer vorzüglich. Es hat sich erwiesen, dass das nachträgliche Hineinschmeizen von Polymer bei liegendem Osteosynthesematerial oder liegenden Prothesen zu ähnlich stabilen Verhältnissen führt.

### Beispiel 10 (Verbundosteosynthese)

Im Rahmen einer Schenkelhalsfraktur bei Osteoporose wurde durch den Schenkelhals eine dynamische Hüftschraube implantiert, welche folgendermassen modifiziert war: innen mit einer zusätzlichen Längsbohrung von 3mm Durchmesser versehen und an der Spitze wo sich das Gewinde befindet mit 10 radiären Löchern von 1 mm Durchmesser versehen, welche eine Kommunikation von zentraler Bohrung und dem Knochen ermöglichen. In diese zentrale Bohrung wurde ein, wie in Beispiel 9 gefertigter Pin von 2.9mm Durchmesser eingeführt und hinten unter mit Licht beaufschlagt. Unter Lichteinwirkung konnte so der Pin im Innern der Schraube geschmolzen werden und das verflüssigte Polymer drang durch die Löcher nach aussen In den Knochen und schuf so eine Augmentation des Knochens in welchem das Implantat hält. Nach aushärten des Polymers (2 Min.) war die Schraube belastungsstabil.

### Beispiel 11 (Memoryeffekt)

Ein partiell lichtabsorbierender Knochenanker wird mit innerer Vorspannung mittels Spritzguss hergestellt (amorphes PLA). In der nun vorhandenen erkalteten Form ist der Anker gerade (Länge 10mm, Durchmesser 3mm). Mit durch eine Öse im oberen Drittel des Ankers durchgefädelten Faden wird der Anker unter sanftem Druck in ein vorgebohrtes Loch am Aussenknöchel gestossen. Unter Wärmeeinwirkung durch das aufgebrachte Licht wird die Relaxation des Ankers eingeleitet und er verbiegt sich. Dadurch verkellt sich der Anker im Knochenloch und findet mechanischen Halt. Der Faden am Anker ist so nach 30 Sekunden belastbar und kann für eine Bankrekonstruktion verwendet werden.

### Beispiel 12 (Nagelverriegelung)

Ein Femurmarknagel wird zur Osteosynthese in den Femur eingebracht. Distal war bei dieser 86jährigen Patientin der Knochen aber zu weich für eine Verriegelung, daher hat der Operateur ein 4mm Loch von lateral durch die Kortikalis zum Nagel hin gebohrt. Ein 3.5mm Pin wurde durch das Loch zum Nagel geschoben. Nun wurde der Pin mit Licht beaufschlagt und in den Markkanal geschoben, wodurch er kontinuierlich am Nagel wegschmolz und den hohlen Markkanal auffüllte und dabei den Nagel einbettete. Damit sich das Implantatmaterial gut in der Markhöhle verteilte wurde eine relativ hohe Energie gewählt (70 Watt) und ein Polymer mit hoher Wärmekapazität gewählt, damit es sich nicht zu schnell abkühlt und erstarrt. Nach Ausschalten des Lichts war der Nagel sicher Im Zentrum des Femurs fixiert. Die Erfindung und Weiterbildungen der Erfindung werden im Folgenden anhand der teilweise schematischen Darstellungen mehrerer Ausführungsbeispiele noch näher erläutert.

Es zeigen:
Fig. 1 einen Längsschnitt durch eine Ausführungsform des erfindungsgemässen medizinischen
   Implantates;
Fig. 2a einen Querschnitt durch eine andere, als Zahnimplantat ausgebildete Ausführungsform des erfindungsgemässen medizinischen Implantates vor dem Schmelzvorgang;
Fig. 2b einen Querschnitt durch die Ausführungsform gemäss Fig. 3a nach erfolgter Implantation;
Fig. 3a eine Ansicht einer anderen Ausführungsform des erfindungsgemässen medizinischen Implantates;
Fig. 3b eine Ansicht der Ausführungsform gemäss Fig. 3a nach erfolgter Implantation;
Fig. 4a eine Ansicht einer weiteren Ausführungsform des erfindungsgemässen medizinischen Implantates;
Fig. 4b eine Ansicht der Ausführungsform gemäss Fig. 4a nach erfolgter Implantation;
Fig. 5a eine Ansicht einer anderen Ausführungsform des erfindungsgemässen medizinischen Implantates;
Fig. 5b eine Ansicht der Ausführungsform gemäss Fig. 5a nach erfolgter Implantation;
Fig. 6a einen Schnitt durch wiederum eine andere Ausführungsform des erfindungsgemässen medizinischen Implantates;
Fig. 6b einen Schnitt durch die Ausführungsform gemäss Fig. 6a nach erfolgter Implantation;
Fig. 7a einen Schnitt durch wiederum eine weitere Ausführungsform des erfindungsgemässen medizinischen Implantates;
Fig. 7b einen Schnitt durch die Ausführungsform gemäss Fig. 7a nach erfolgter Implantation;
Fig. 8 einen Schnitt durch wiederum eine weitere Ausführungsform des erfindungsgemässen medizinischen Implantates;
Fig. 9a einen Schnitt durch wiederum eine andere Ausführungsform des erfindungsgemässen medizinischen Implantates;
Fig. 9b einen Schnitt durch die Ausführungsform gemäss Fig. 9a während Implantation;
Fig. 9c einen Schnitt durch die Ausführungsform gemäss den Fig. 9a und 9b nach erfolgter Implantation;
Fig. 10 einen Schnitt durch eine weitere Ausführungsform des erfindungsgemässen medizinischen Implantates;
Fig. 11 einen Schnitt durch wiederum eine weitere Ausführungsform des erfindungsgemässen medizinischen Implantates;
Fig. 12 einen Schnitt durch eine andere Ausführungsform des erfindungsgemässen medizinischen Implantates;
Fig. 13a einen Schnitt durch wiederum eine andere Ausführungsform des erfindungsgemässen medizinischen Implantates;
Fig. 13b einen Schnitt durch die Ausführungsform gemäss Fig. 13a nach erfolgter Implantation; und

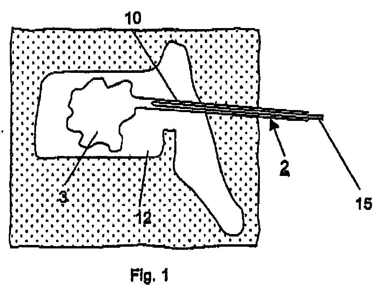

In der In Fig. 1 dargestellten Ausführungsform umfasst das medizinische Implantat einen Pin 2 und wird für eine Anwendung in der Vertebroplastik verwendet (Beispiel 9). Durch ein vorgebohrtes Loch 10 in einem Pedikel eines zu behandelnden Wirbelkörpers 12 wird von dorsal noch ohne Zufuhr von Licht ein Pin 2 aus Poly-L-co-D,L-lactid eingeführt.

Nach Einführung des Pins 2 wird das Licht eingeschaltet und der Pin 2 mit dem angeschlossenen Lichtleiter 15 in den Wirbelkörper 12 geschoben. Unter Nachschieben des Pins 2 kann so eine Füllung 3 des Wirbelkörpers 12 mit Poly-L-co-D, L-lactid erreicht werden. Nach 2 Minuten Auskühlen ist der Wirbelkörper belastungsstablil und schmerzfrei.

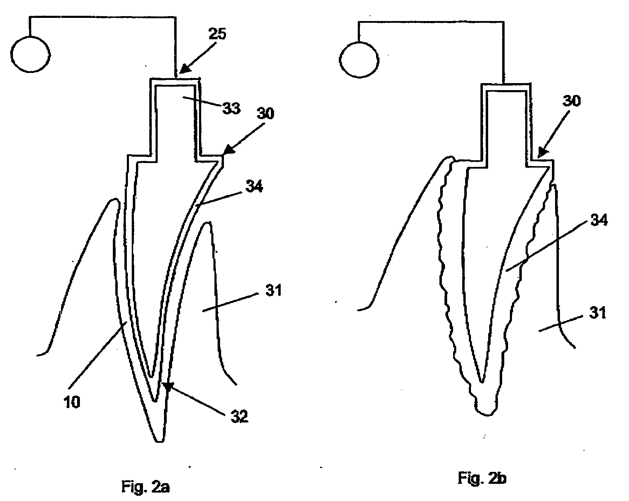

Die in den Fig. 2a und 2b dargestellte Ausführungsform umfasst ein Zahnimplantat 30 aus Titan, welches mit einer Schicht 34 aus amorphem Poly-D, L-lactid umgeben ist. Das vom distalen Ende 32 weggewandte, beschichtete Ende 33 wird mit Licht 25 beaufschlagt. Das Zahnimplantat 30 wird in das mit Untermass vorgebohrte Loch 10 gesetzt und das Licht eingeschaltet (Fig. 2a). Sobald die Lichtabsorption in der Schicht 34 erfolgt, erweicht die Schicht 34 und das Zahnimplantat 30 kann nun mittels Druck in die Tiefe des Lochs 10 gedrückt werden. Beim Einpressen des Zahnimplantates 30 in das Loch 10 wird der die Schicht 34 bildende Thermoplast in die Zwischenräume im Knochen 31 gepresst, so dass eine mechanische Verbindung zwischen dem Zahnimplantat 30 und dem Knochen 31 geschaffen wird. Das Erstarren des Polymers, d.h. der Schicht 34 im Knochen 31 führt zu einer primären, belastungsstabilen Verbindung zwischen Knochen 31 und Zahnimplantat 30 (Fig. 2b).

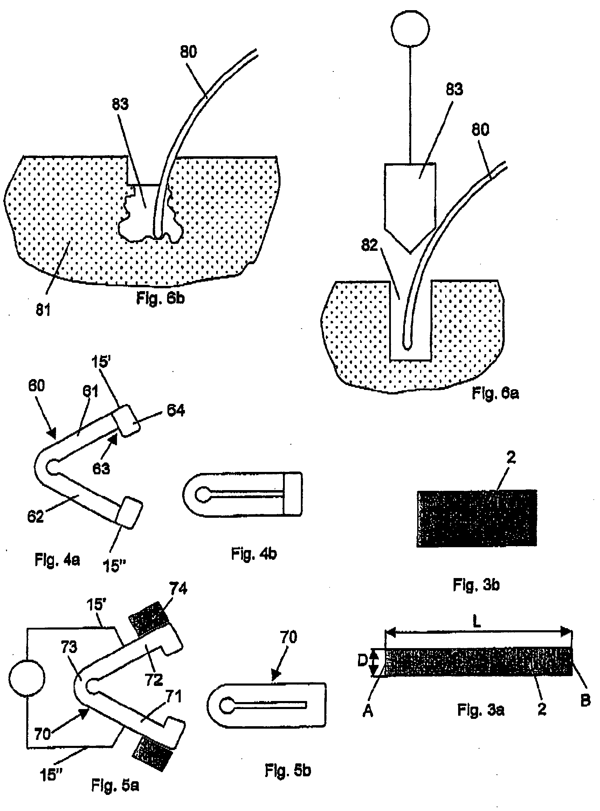

In den Fig. 3a und 3b ist eine weitere Ausführungsform dargestellt, wobei der Pin 2 durch ein geeignetes Herstellungsverfahren, z.B. mittels Spritzgiessen, mit einer Eigenspannung versehen wird und im erkalteten Zustand eine Länge L und einen Durchmesser D aufweist (Fig. 3a). Durch das Erwärmen des gesamten Pins 2 mittels Strahlungszufuhr an einem der Enden A1B relaxiert der Thermoplast und der Pin 2 wird kürzer und nimmt im Durchmesser zu (Fig. 3b), was zu einer Fixation im oder am umgebenden Gewebe führt.

in der in den Fig. 4a und 4b dargestellten Ausführungsform ist das medizinische Implantat als Clip 60 ausgebildet. Der Clip 60 ist U-förmig ausgebildet und umfasst zwei Arme 61,62 deren freie Enden 63 je ein aus Poly-L-co-D, L-lactid bestehendes Element 64 umfassen. Diese gegenüber den Armen 61 ,62 dickeren vnd eingefärbten Elemente 64 werden mittels Lichtleitern 16', 15" mit Strahlungsenergie beaufschlagt (Fig. 4a). Nach dem Einschalten des Lichts wird der Clip 60 zusammengepresst, d.h. die beiden Elemente 64 werden aneinander gepresst. Durch Lichtabsorption werden die beiden Elemente 64 erwärmt und erweichen an ihren aneinander anliegenden Kontaktstellen und können so durch ein Aufbringen von Druck zusammengefügt und durch Verschmelzen miteinander verbunden werden (Fig. 4b).

Der in den Fig. 5a und 5b dargestellte Clip 70 unterscheidet sich von dem in den Fig. 4a und 4b dargestellten Clip nur darin, dass der Clip 70 einstückig aus Poly-L-co-D, L-laktid hergestellt ist. Die Arme 71 ,72 werden mit einer Klemme 74 gegriffen, über je einen Lichtleiter 15', 15" mit Licht beaufschlagt und zusammengedrückt. Durch die Strahlungsenergie erweicht das die Arme 71 ,72 verbindende Scharnier 73 und erlaubt ein Verbiegen des Clips 70. Beim Aufeinandertreffen der dem Scharnier 73 abgewandten Enden der Arme 71,72 erfolgt durch Aufschmelzen das gewünschte Verbinden der beiden Arme 71,72 an den gegenüber den Armen 71 ,72 verdickten Enden.

In der in den Fig. 6a und 6b dargestellten Ausführungsform umfasst das medizinische Implantat einen Faden 80, welcher aus einem Material mit hohem Schmelzpunkt besteht, und einen Anker 83 aus einem Polymer. Der Faden 80 soll am Knochen 81 fixiert werden, um mit dem Faden 80 z.B. eine Sehne oder ein anderes Knochenstück zu halten. Dafür wird ein Loch 82 mit einem Durchmesser von 3mm 15mm tief In den Knochen 81 gebohrt. In dieses Loch 82 im Knochen 81 wird der Faden 80 eingeführt. Anschliessend wird auf das Loch 82 ein Anker 83 aufgesetzt, welcher im Durchmesser etwas dicker ist als das Loch 82. Analog zum Beispiel 1 wird auch hier der Anker 83 mittels Licht mit Strahlungsenergie beaufschlagt und nach Aufweichen durch das Licht in den Knochen 81 gedrückt. Nach Ausschalten des Lichts, erstarrt das leitfähige Polymer und der Anker 83 ist zusammen mit dem Faden 80 im Knochen 81 fixiert.

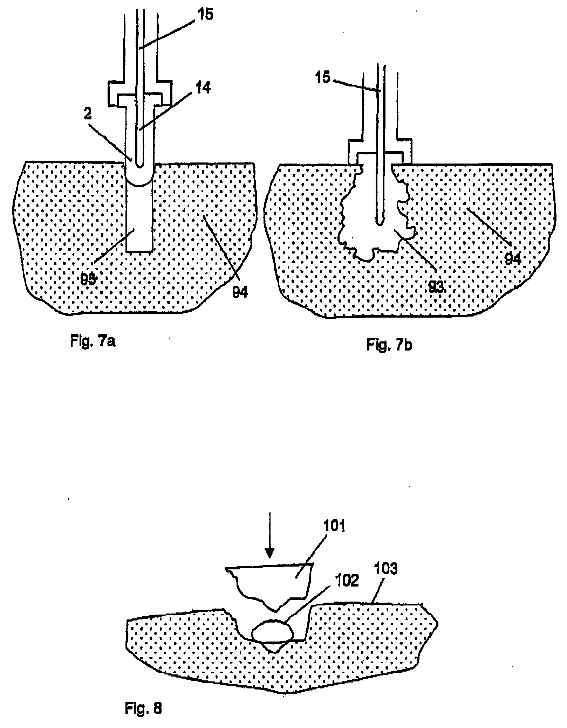

In den Fig. 7a, 7b ist eine Ausführungsform des medizinischen Implantates dargestellt, welche zum Füllen von Defekten an Knochen 94 geeignet ist. Analog zur Ausführungsform gemäss Fig. 1 wird ein Pin 2 verwendet, welcher einen zentralen, an der Spitze des Pins 2 geschlossenen Hohlraum 91 zur Aufnahme eines Lichtleiters 15 aufweist. Der Lichtleiter 15 kann nach dem Verschmelzen des Pins 2 wieder entfernt werden oder auch aus einem resorbierbaren Material gefertigt sein. Zum Füllen z.B. eines Tibiakopfdefekts wird bei einem Patienten mit Tibiakopffraktur ein 4mm Loch 95 von ventral durch die Kortikalis zum Defekt hin gebohrt (Länge 2cm). Durch dieses Loch 95 wird darauf der Pin 2 zusammen mit dem Lichtleiter 15 in die Markhöhle und den spongiösen Raum des Knochens unter Lichtanwendung geschoben, und so wie bei einer Verbundosteosynthese durch Verschmelzen des Pins 2 zur einer Füllung 93 ein stabiler Knochen geschaffen. Die danach in diese Füllung 93 eingebrachten Schrauben (nicht gezeichnet) halten im zuvor geschmolzenen und nachher erstarrten Polymer vorzüglich.

In Fig. 6 ist eine Ausführungsform dargestellt, In welcher das Polymer des medizinischen Implantates als Perle 102 ausgebildet ist. Diese Perle 102 kann in den Hohlraum eingefügt werden, welcher beim Herausbrechen eines Knochenfragmentes 101 aus einem Knochen 103 entsteht. Das Einpassen des Knochenfragments 101 in den Hohlraum und Verbinden des Knochenfragments 101 mit dem Knochen 103 mittels Schmelzen der Perle 102 und Pressen des Polymers in die Zwischenräume im Knochenfragment 101 und im Knochen 103 erfolgt durch Bestrahlen der Perle 102 mit Licht, welche sich in der Folge erwärmt und verformen kann.

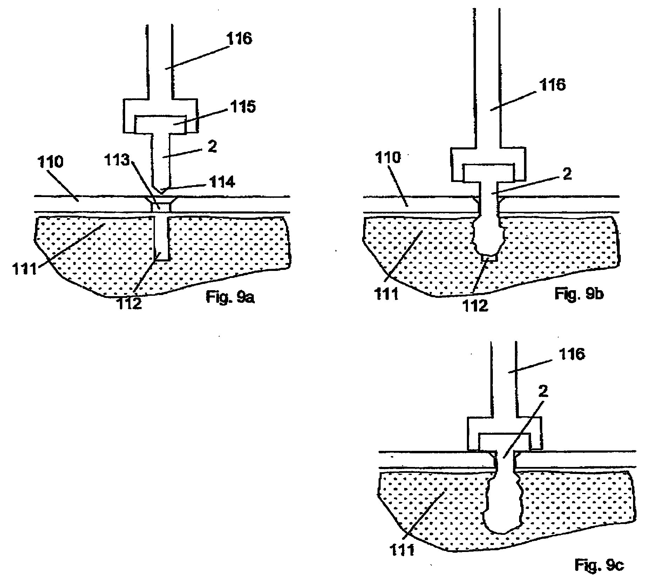

Die in den Fig. 9a - 9c dargestellte Ausführungsform umfasst einen aus Poly-D, L-Lactid hergestellten Pin 2, welcher zur Fixation einer Knochenplatte 110 an einem Knochen 111 geeignet ist. Die Knochenplatte 110 ist eine resorbierbare Osteosyntheseplatte mit einer Dicke von 1 mm aus dem gleichen Material. Zur Fixation einer Fraktur wird die Knochenplatte 110 auf die zu fixierenden Knochenfragmente aufgebracht und die für deren Fixation am Knochen 111 benötigten Löcher 112 in den Knochen 111 gebohrt. In diesem Beispiel handelt es sich um eine Knochenplatte 110 mit Schraubenlöchern 113 für 2.0 mm Schrauben. Die In den Knochen 111 gebohrten Löcher 112 haben einen Durchmesser von 1.5 mm. Der Pin 2 hat einen Durchmesser von 2.0 mm wird mit seinem hinteren, im Durchmesser vergrösserten Kopf 115 an ein Instrument 116 angebracht (Fig. 9a).

Der Lichtleiter kann koaxial durch eine Zentralbohrung (nicht gezeichnet) im Instrument 116 durchgerührt werden.

Der Pin 2 wird mit seiner in den Knochen 111 einzubringenden Spitze 114 durch das Schraubenloch 113 in der Knochenplatte 110 geführt und auf das im Knochen 111 vorgebohrte Loch 112 aufgesetzt und mit Licht beaufschlagt. Die Zufuhr von Lichtenergie durch den Pin 2 erwärmt diesen. Durch Druck auf das Instrument 15 wird der Pin 2 in das in den Knochen 111 vorgebohrte Loch 112 geschoben und das thermoplastische Material fliesst in die zugänglichen intertrabekulären Zwischenräume im spongiösen Knochen (Fig. 9b). Nach Ausschalten des Lichts kühlt das Polymer wieder ab und erstarrt. Der im Durchmesser gegenüber dem Schraubenloch 113 in der Knochenplatte 110 grössere Kopf 115 des Pins 2 hält nun die Knochenplatte 110 (Fig. 9c).

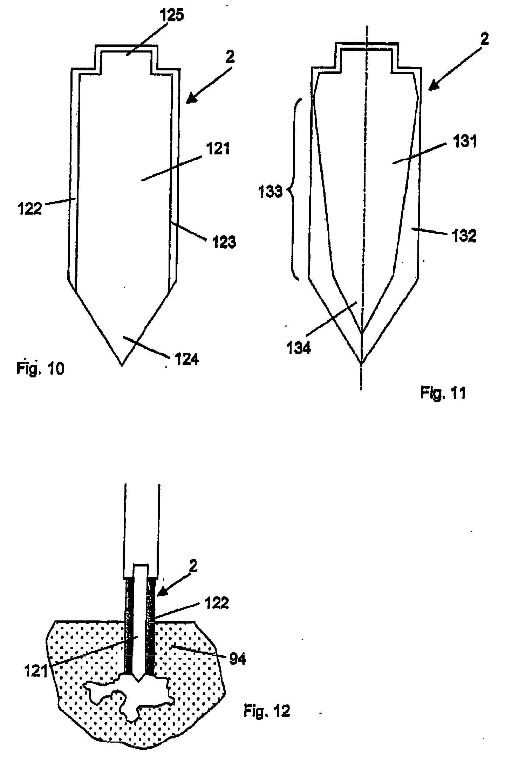

Fig. 10 und 11 zeigen je einen Pin 2, weicher einen Kern 121 ,131 beispielsweise aus einem metallischen Material und eine Beschichtung 122,132 aus Poly-D, L-lactid umfasst. Die Beschichtung 122 in Fig. 10 ist hülsenförmig ausgebildet und erstreckt sich über den zylindrischen Teil 123 und das hintere Ende 125 des Pins 2. Die Spitze 124 des Pins 2 ist ohne Beschichtung ausgebildet. Die Beschichtung 132 in Fig. 11 ist nur partiell an einem vorderen Abschnitt 133 des Pins 2 angebracht, und umschliesst den sich verjüngenden Abschnitt 133 des Pins 2 inklusive dessen Spitze 134 und das hintere Ende. Ein gemäss den Fig. 10 oder 11 ausgebildeter Pin 2 ermöglicht eine selektive Erwärmung eines Thermoplasten um eine Verformung zu erreichen.

Flg. 12 zeigt die Anwendung eines Pins 2 gemäss Fig. 10 bei der in den Fig. 7a und 7b beschriebenen Füllung eines Defektes an einem Knochen 94.

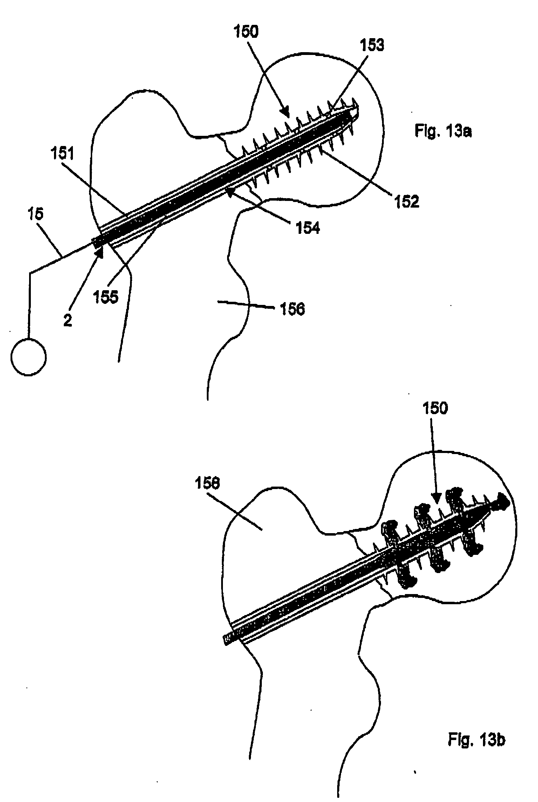

Fig. 13a und 13b zeigen eine Ausführungsform, worin das medizinische Implantat eine dynamische Hüftschraube 150 und einen aus einem Polymer bestehenden Pin 2 umfasst. Die dynamische Hüftschraube 150 hat einen hohlen Schaft 151 mit einem Knochengewinde 152 am vorderen, bis in den Hoftgelenkkopf reichenden Ende. Im Bereich des Knochengewindes 152 sind radiale Perforationen 153 angeordnet, welche den Schaft 151 zwischen dessen zentralem Hohlraum 154 und seiner Peripherie radial durchdringen. Der Hohlraum 154 ist ausser im Bereich der Perforationen 153 mit einer isolierenden Beschichtung 155 versehen. Die dynamische Hüftschraube 150 wird im Rahmen einer Schenkelhalsfraktur bei Osteoporose durch den Schenkelhals implantiert. In den zentralen Hohlraum 154 wird ein, wie unter Beispiel 9 beschrieben, isolierter Pin 2 von 2.9 mm Durchmesser eingeführt und an seinem hinteren, dem Knochengewinde 152 der dynamischen Hüftschraube 150 entgegengesetzten Ende mittels eines Lichtleiters 15 mit Licht beaufschlagt. Unter Lichtabsorption schmilzt so der Pin 2 im Innern der Hüftschraube 150 und das verflüssigte Polymer dringt durch die Perforationen 153 nach aussen in den Knochen 156 und schafft so eine Augmentation des Knochens 156 in welchem das Implantat hält. Nach Aushärten des Polymers ist die Hüftchraube 150 belastungsstabil (Fig. 13b).

## Patentansprüche

1. Medizinisches Implantat zur Osteosynthese, welches ein oder mehrere körperverträgliche Polymere umfasst und welches mit einer Eigenspannung oder einer inneren Vorspannung versehen ist, welche bei einer Erwärmung relaxieren kann,
**dadurch gekennzeichnet, dass**
das eine oder die mehreren körperverträglichen Polymere einen Farbstoff enthalten, so dass das eine oder die mehreren körperverträglichen Polymere mittels elektromagnetischer Bestrahlung erwärmbar und erweichbar sind.

2. Medizinisches Implantat nach Anspruch 1, **dadurch gekennzeichnet, dass** das Implantat mindestens zum Teil aus einem zu erwärmenden Polymer besteht, welches eine minimale molare Wärmekapazität cₚ von 1 ,6 kJ/kmolK, vorzugsweise von 2,2 kJ/kmolK aufweist.

3. Medizinisches Implantat nach Anspruch 1 oder 2, **dadurch gekennzeichnet, dass** das Implantat mindestens zum Teil aus einem zu erwärmenden Polymer besteht, welches eine maximale molare Wärmekapazität cₚ von 2,9 kJ/kmolK, vorzugsweise von 2,5 kJ/kmolK aufweist.

4. Medizinisches Implantat nach einem der Ansprüche 1 bis 3, **dadurch gekennzeichnet, dass** das Polymer derart ausgewählt ist, dass die Erweichung unterhalb einer Erwärmungstemperatur von 250°C stattfindet.

5. Medizinisches Implantat nach einem der Ansprüche 1 bis 4, **dadurch gekennzeichnet, dass** ausser dem Polymer selbst keine anderen Bauteile des Implantats für die Erwärmung des Implantats vorgesehen sind.

6. Medizinisches Implantat nach einem der Ansprüche 1 bis 5, **dadurch gekennzeichnet, dass** es Mittel zur Befestigung eines Lichtleiters mit mindestens einer lichtdurchlässigen Faser umfasst.

7. Medizinisches Implantat nach einem der Ansprüche 1 bis 6, **dadurch gekennzeichnet, dass** das zu erwärmende und zu erweichende Polymer optisch und/oder mechanisch isotrop ist.

8. Medizinisches Implantat nach einem der Ansprüche 1 bis 7, **dadurch gekennzeichnet, dass** das zu erwärmende und zu erweichende Polymer optisch und/oder mechanisch anisotrop ist.

9. Medizinisches Implantat nach einem der Ansprüche 1 bis 8, **dadurch gekennzeichnet, dass** das zu erwärmende und zu erweichende Polymer ein thermoplastisches Material ist, welches aus den folgenden Gruppen gewählt ist: Poly-alpha-hydroxyester, Polyorthoester, Polyanhydrid, Polyphosphazenes, Poly(propylenfumarat), Polyesteramid, Polyethylenfumarat, Polylaktid, Polyglykolid, Polycaprolacton, Trimethylencarbonat, Polydioxanon, Polyhydroxybutyrat, sowie deren Copolymere und Gemische.

10. Medizinisches Implantat nach einem der Ansprüche 1 bis 9, **dadurch gekennzeichnet, dass** das zu erwärmende und zu erweichende Polymer eine matte oder optisch streuende offenporige Struktur aufweist.

11. Medizinisches Implantat nach einem der Ansprüche 1 bis 10, **dadurch gekennzeichnet, dass** das zu erwärmende und zu erweichende Polymer kapillare Kanäle aufweist.

12. Medizinisches Implantat nach einem der Ansprüche 1 bis 11, **dadurch gekennzeichnet, dass** das zu erwärmende und zu erweichende Polymer hydrophile Eigenschaften aufweist.

13. Medizinisches Implantat nach einem der Ansprüche 1 bis 12, **dadurch gekennzeichnet, dass** das zu erwärmende und zu erweichende Polymer eine Mischung aus mindestens zwei verschiedenen körperverträglichen, thermoplastischen Materialien umfasst.

14. Medizinisches Implantat nach einem der Ansprüche 1 bis 13 zur Fixierung von Knochen oder Knochenfragmenten.

## Claims

1. A medical implant for osteosynthesis, which comprises one or more biocompatible polymers and which is furnished with internal stress or an internal prestress that relaxes when heated,
**characterized in that**
the one or more biocompatible polymers contain a dye, so that the one or more biocompatible polymers can be heated and softened by means of electromagnetic radiation.

2. The medical implant according to claim 1, **characterized in that** the implant is made at least in part of a polymer to be heated that has a minimum molar heat capacity C_{P} of 1.6 kJ/kmol K, preferably of 2.2 kJ/kmol K.

3. The medical implant according to claim 1 or 2, **characterized in that** the implant is made at least in part of a polymer to be heated that has a maximum molar heat capacity C_{P} of 2.9 kJ/kmol K, preferably of 2.5 kJ/kmol K.

4. The medical implant according to any one of claims 1 to 3, **characterized in that** the polymer is selected such that softening occurs below a heating temperature of 250° C.

5. The medical implant according to any one of claims 1 to 4, **characterized in that** with the exception of the polymer itself, no other components of the implant are provided for heating the implant.

6. The medical implant according to any one of claims 1 to 5, **characterized in that** it comprises means for attaching a light guide having at least one translucent fiber.

7. The medical implant according to any one of claims 1 to 6, **characterized in that** the polymer to be heated and softened is optically and/or mechanically isotropic.

8. The medical implant according to any one of claims 1 to 7, **characterized in that** the polymer to be heated and softened is optically and/or mechanically anisotropic.

9. The medical implant according to any one of claims 1 to 8, **characterized in that** the polymer to be heated and softened is a thermoplastic material, selected from the following groups: poly alpha-hydroxy esters, poly ortho esters, polyanhydride, polyphosphazenes, poly(propylene fumarate), polyester amide, polyethylene fumarate, polylactide, polyglycolide, polycaprolactone, trimethylene carbonate, polydioxanone, polyhydroxybutyrate, and copolymers and mixtures thereof.

10. The medical implant according to any one of claims 1 to 9, **characterized in that** the polymer to be heated and softened has a matte or optically diffusive, openpored structure.

11. The medical implant according to any one of claims 1 to 10, **characterized in that** the polymer to be heated and softened has capillary channels.

12. The medical implant according to any one of claims 1 to 11, **characterized in that** the polymer to be heated and softened has hydrophilic properties.

13. The medical implant according to any one of claims 1 to 12, **characterized in that** the polymer to be heated and softened comprises a mixture of at least two different biocompatible, thermoplastic materials.

14. The medical implant according to any one of claims 1 to 13 for securing bones or bone fragments.

## Revendications

1. Implant médical destiné l'ostéosynthèse, qui comprend un ou plusieurs polymères biocompatibles et qui est pourvu d'une contrainte propre ou d'une précontrainte interne qui peut se détendre lors d'un échauffement,
**caractérisé en ce que**
un polymère ou les plusieurs polymères organiques biocompatibles contiennent un colorant de sorte que le ou les plusieurs polymères biocompatibles peuvent être chauffés et ramollis au moyen d'un rayonnement électromagnétique.

2. Implant médical selon la revendication 1, **caractérisé en ce que** ledit implant est constitué au moins en partie d'un polymère à chauffer qui présente une capacité calorifique molaire minimale cₚ de 1,6 kJ/kmolK, de préférence de 2,2 kJ/kmolK.

3. Implant médical selon la revendication 1 ou 2, **caractérisé en ce que** l'implant est constitué au moins en partie d'un polymère à chauffer qui présente une capacité calorifique molaire maximale cₚ de 2,9 kJ/kmolK, de préférence de 2,5 kJ/kmolK.

4. Implant médical selon l'une des revendications 1 à 3, **caractérisé en ce que** le polymère est choisi de telle sorte que le ramollissement se produit au-dessous d'une température de chauffage de 250°C.

5. Implant médical selon l'une des revendications 1 à 4, **caractérisé en ce qu'**en plus du polymère lui-même aucun autre composant de l'implant n'est prévu pour le chauffage de l'implant.

6. Implant médical selon l'une des revendications 1 à 5, **caractérisé en ce qu'**il comprend des moyens de fixation d'un guide de lumière qui comporte au moins une fibre de transmission de lumière.

7. Implant médical selon l'une des revendications 1 à 6, **caractérisé en ce que** le polymère à chauffer et à ramollir est optiquement et/ou mécaniquement isotrope.

8. Implant à usage médical selon l'une des revendications 1 à 7, **caractérisé en ce que** le polymère à chauffer et à ramollir est optiquement et/ou mécaniquement anisotrope.

9. Implant médical selon l'une des revendications 1 à 8, **caractérisé en ce que** le polymère à chauffer et à ramollir est une matière thermoplastique qui est choisie dans le groupe suivant : poly-alpha-hydroxy-ester, polyorthoester, polyanhydride, polyphosphazènes, fumarate de polypropylène, amide de polyester, fumarate de polyéthylène, polylactide, polyglycolide, polycaprolactone, carbonate de triméthylène, polydioxanone, polyhydroxybutyrate ainsi que leurs copolymères et mélanges.

10. Implant à usage médical selon l'une des revendications 1 à 9, **caractérisé en ce que** le polymère à chauffer et ramollir comporte une structure mate ou optiquement diffusante à pores ouverts.

11. Implant à usage médical selon l'une des revendications 1 à 10, **caractérisé en ce que** le polymère à chauffer et à ramollir comporte des conduits capillaires.

12. Implant à usage médical selon l'une des revendications 1 à 11, **caractérisé en ce que** le polymère à chauffer et ramollir a des propriétés hydrophiles.

13. Implant à usage médical selon l'une des revendications 1 à 12, **caractérisé en ce que** le polymère à chauffer et à ramollir comporte un mélange d'au moins deux matières thermoplastiques biocompatibles différentes.

14. Implant à usage médical selon l'une des revendications 1 à 13 destiné à la fixation d'os ou de fragments d'os.
